# EUROPÄISCHE PATENTANMELDUNG

(11) **EP 2 338 537 A2**
(43) Veröffentlichungstag der Anmeldung: **29.06.2011**
(21) Anmeldenummer: 10192875.2
(22) Anmeldetag: 29.11.2010
(51) Int. Cl.: A61L 31/08, A61L 31/16

(54) **Aptamer beschichtetes Implantat, Herstellverfahren und Verwendungen**

(30) Priorität: 21.12.2009 US 288361 P
(71) Anmelder: Biotronik VI Patent AG, 6341 Baar (CH)
(72) Erfinder: Borck, Alexander, 91086, Aurachtal (DE)
(74) Vertreter: Lindner-Vogt, Karin L.

(57) **Zusammenfassung**

Die vorliegende Erfindung betrifft ein mit Aptamer beschichtetes Implantat, wobei das Aptamer eine aktivierende Wirkung gegenüber dem αᵥβ₃-Integrin-Rezeptor aufweist, zur Verbesserung der Endothelzelladhäsion an das Implantat, dessen Herstellverfahren sowie Verwendungen gemäß der unabhängigen Ansprüchen.

## Beschreibung

Die vorliegende Erfindung betrifft ein mit Aptamer beschichtetes Implantat, wobei das Aptamer eine aktivierende Wirkung gegenüber dem (αᵥβ₃-Integrin-Rezeptor aufweist, zur Verbesserung der Endothelzelladhäsion an das Implantat, dessen Herstellverfahren sowie Verwendungen gemäß der unabhängigen Ansprüchen.

Implantate sind Stoffe oder Teile, die zur Erfüllung bestimmter Ersatzfunktionen für einen begrenzten Zeitraum oder auf Lebenszeit in den menschlichen oder tierischen Körper eingebracht werden. Im Gegensatz zu Transplantaten bestehen Implantate aus künstlichem Material (Alloplastik). Es wird häufig nach medizinischen, plastischen oder funktionellen Implantaten unterschieden.

Medizinische Implantate haben die Aufgabe, Körperfunktionen zu unterstützen oder zu ersetzen. Je nach Funktion werden sie auch als implantierbare Prothese bezeichnet. Bekannte Vertreter sind z. B. (Elektroden für) Herzschrittmacher, Herzimplantate, wie beispielsweise Herzklappen und Shunts, (koronare und periphere) Stents, Neurostents.

Stents im Allgemeinen sind endovaskuläre (periphere oder koronare) Prothesen bzw. Implantate, die beispielsweise zur Behandlung von Stenosen, aber auch zur Behandlung von Aneurysmen bekannt sind. Stents weisen grundsätzlich eine Tragstruktur auf, die geeignet ist, die Wand eines Gefäßes in geeigneter Weise abzustützen, um so das Gefäß zu weiten bzw. ein Aneurysma zu überbrücken. Stents werden dazu in einem komprimierten Zustand in das Gefäß eingeführt, dann an dem zu behandelnden Ort aufgeweitet und gegen die Gefäßwand gedrückt. Dieses Aufweiten kann beispielsweise mit Hilfe eines Angioplastie-Ballons, aufgebracht auf einen einführbaren Katheter, erfolgen. Alternativ sind auch selbstexpandierende Stents bekannt. Diese sind beispielsweise aus einem superelastischen Metall, wie Nitinol, aufgebaut.

Stents werden derzeit in zwei Grundtypen eingeteilt, die dauerhaften Stents und degradierbaren Stents. Dauerhafte Stents sind so ausgestaltet, dass sie im Gefäß für einen unbestimmten Zeitraum verbleiben könnten. Degradierbare Stents hingegen werden über einen vorbestimmten Zeitraum hinweg in einem Gefäß abgebaut.

Im Bereich der Herz-Kreislauf-Erkrankungen stellen minimalinvasive Therapieformen wie beispielsweise die Aufweitung und Stabilisierung stenosierter Koronargefäße mittels der Perkutanen Transluminalen Coronarangioplastie (PTCA) in Verbindung mit der Stentimplantation eine sich immer weiter etablierende Behandlungsmethode dar. Als Spätkomplikation ist hier neben einer Wiederverengung des Gefäßes nach PTCA oder StentImplantation (sogenannte In-Stent-Restenose, (ISR)) und einer Gewebsentzündung im Bereich der Behandlung mittels PTCA und/oder der Stentimplantation vor allem das Risiko einer Thromboembolie zu nennen. Vor diesem Hintergrund werden insbesondere Stents im Stand der Technik bereitgestellt, die mit Wirkstoffen beschichtet sind (sogenannte "Drug Eluting Stents", DES), die einer oder mehreren Spätkomplikationen entgegenwirken sollen. Allerdings können DES, die beispielsweise der Intimahyperplasie entgegenwirken, als unerwünschte Nebenwirkungen einerseits Nekrotisierungen des Gefäßgewebes im Bereich der Stentimplantation und andererseits Langzeitschäden, wie Spätthrombosen zeigen.

Der physikalischen Verankerung von Zellen dienen vornehmlich die Integrine, welche als heterodimere Glykoproteine auch zur bidirektionalen Signaltransduktion durch die Zellwand genutzt werden. Durch die Bindung eines Liganden an das Integrin wird ein Signal in die Zelle übertragen, woraus eine Umorientierung des Cytoskeletts resultiert. Der als "outside-in signaling" bezeichnete Prozess kann die Veränderung der Zellform, die Migration oder die verankerungsabhängige Proliferation sowie die Gewebsorganisation steuern. Der αᵥβ₃-Integrin-Rezeptor besitzt viele unterschiedliche Ligandenist insbesondere auf Endothelzellen, reifen, knochenresorbierenden Osteoklasten, abwandernden glatten Muskelzellen, Tumorzellen, Thrombozyten, Fibroblatsten, aktivierte Makrophagen/Monozyten, neutrophile Granulozyten zu finden. Der αᵥβ₃-Integrin-Rezeptor spielt dementsprechend insbesondere eine wichtige Rolle bei der Atherogenese, der Knochenresproption, in der Neovaskularisation/Angiogenese und bei Entzündungsgeschehen.

Aus "Funktionalisierung künstlicher Oberflächen mit Integrinliganden zur Stimulierung integrinvermittelter Zelladhäsion" (Dissertation, Jörg Auernheimer, Technische Universität München, Department Chemie, 2005) sind Verfahren bekannt, bei denen künstliche Oberflächen insbesondere mit (zyklischen) RGD-Peptiden, die Arginin-Glycin-Asparagin umfassen, als Integrinliganden (insbesondere αᵥβ₃-Integrin-Rezeptor) beschichtet werden, um eine integrinvermittelte Zelladhäsion zu erreichen. Alternative Ansätze sehen eine Beschichtung mit Antikörpern, wie beispielsweise Anti CD34 und Anti CD133 vor.

In WO 2004/055153 werden Implantate beschrieben, die mit Aptameren beschichtet sind, um eine Adhäsion von biologischem Material an das Implantat zu erreichen. Aptamere haben gegenüber Proteinen den Vorteil, dass sie u.a. stabiler gegenüber Sterilisierungsverfahren sind und spezifischer und/oder stärker an Zielmoleküle binden können.

Aus anderen Veröffentlichungen, wie beispielsweise "Aptamer selection for the inhibition of cell adhesion with fibronectin as target", Ogawa, A. et al, Bioorganic & Medicinal Chemistry Letters 14 (2004) 4001 - 4004; "Targeted inhibition of aphavbeta3 integrin with an RNA aptamer impairs endothelial cell growth and survival", Mi, J. et al, Biochem Biophys Res Commun 2005, 338:956-963, sind allerdings nur Aptamere bekannt, die zwar an den αᵥβ₃-Integrin-Rezeptor binden, jedoch eine (Endothel)-Zelladhäsion inhibieren.

Die Aufgabe der vorliegenden Erfindung besteht nun darin, ein Implantat bereitzustellen, dessen Biokompatibilität erhöht ist und dabei vorzugsweise die Einheilung des Implantat in das Gewebe zu erhöhen und/oder das Risiko von Spätkomplikationen zu reduzieren.

Die vorliegende Aufgabe wird durch die erfindungsgemäßen Gegenstände der unabhängigen Ansprüche gelöst.

Demnach betrifft eine erste erfindungsgemäße Ausgestaltung ein Implantat für einen menschlichen oder tierischen Körper, dadurch gekennzeichnet, dass ein Implantatgrundkörper eine vollständige oder teilweise Beschichtung mit einem oder mehreren, gleichen oder verschiedenen Aptameren umfasst, wobei das oder die Aptamere eine aktivierende Wirkung gegenüber dem (αᵥβ₃-Integrin-Rezeptor aufweisen.

Eine zweite erfindungsgemäße Ausgestaltung betrifft ein Verfahren zur Herstellung eines erfindungsgemäßen Implantates, dadurch gekennzeichnet, dass das Verfahren folgende Schritte umfasst oder daraus besteht:
a) Bereitstellen eines Implantatgrundkörpers,
b) Bereitstellen eines Beschichtungsmaterials umfassend ein oder mehrere, gleiche oder verschiedene Aptamere, die eine aktivierende Wirkung gegenüber dem αᵥβ₃-Integrin-Rezeptor aufweisen, wobei das Beschichtungsmaterial geeignet ist, das oder die Aptamere unter physiologischen Bedingungen aus der Beschichtung freizusetzen und
c) Aufbringen des Beschichtungsmaterials aus Schritt b) auf mindestens einen Teil der Oberfläche und/oder in Kavität(en) des Implantatgrundkörpers.

Eine dritte erfindungsgemäße Ausgestaltung betrifft ein Verfahren zur Herstellung eines erfindungsgemäßen Implantates, wobei das oder die Aptamere unter physiologischen Bedingungen im Wesentlichen nicht aus der Beschichtung freigesetzt werden können, dadurch gekennzeichnet, dass das Verfahren folgende Schritte umfasst oder daraus besteht:
a) Bereitstellen eines Implantatgrundkörpers,
b) Bereitstellen einer oder mehrerer, gleicher oder verschiedener Ankergruppen,
c) Funktionalisieren des Implantatgrundkörpers aus Schritt a) mit der oder den Ankergruppen aus Schritt b),
d) Bereitstellen eines oder mehrerer, gleicher oder verschiedener Aptamere, die eine aktivierende Wirkung gegenüber dem αᵥβ₃-Integrin-Rezeptoren aufweisen und
e) Binden des oder der Aptamere aus Schritt d) an den oder die funktionalisierten Implantatgrundkörper aus Schritt c).

Eine vierte erfindungsgemäße Ausgestaltung betrifft eine Verwendung eines Aptamers mit aktivierender Wirkung gegenüber dem αᵥβ₃-Integrin-Rezeptor zur Vermittlung von Endothelzelladhäsion an ein Implantat für einen menschlichen oder tierischen Körper.

Eine fünfte erfindungsgemäße Ausgestaltung betrifft eine Verwendung eines oder mehrerer unterschiedlicher Aptamere mit aktivierender Wirkung gegenüber dem αᵥβ₃-Integrin-Rezeptor zur vollständigen oder teilweisen Beschichtung eines Implantatgrundkörpers für einen menschlichen oder tierischen Körper.

Eine sechste erfindungsgemäße Ausgestaltung betrifft eine Verwendung eines oder mehrerer unterschiedlicher Aptamere mit aktivierender Wirkung gegenüber dem αᵥβ₃-Integrin-Rezeptor zur Herstellung eines erfindungsgemäßen Implantats.

Bevorzugte Ausgestaltungen der Erfindung werden in den abhängigen Ansprüchen sowie der nachfolgenden detaillierten Beschreibung dargestellt und sind, sofern für den Fachmann sinnvoll, untereinander kombinierbar.

Der vorliegenden Erfindung liegt die Erkenntnis zugrunde, dass erfindungsgemäß zu verwendende Aptamere aufgrund Ihrer aktivierenden Wirkung gegenüber dem αᵥβ₃-Integrin-Rezeptor spezifisch eine Endothelzelladhäsion an die Implantatoberfläche und damit einhergehenden Ausbildung einer Endothelschicht, die vergleichbar mit gesundem Gewebe ist, vermitteln und so die Biokompatibilität von Implantaten erhöhen. Neben der beschleunigten Einheilung des Implantats kann ein beschleunigtes Bewachsen des Implantats mit Endothelzellen und Ausbilden einer funktionalen, d.h. dem gesunden Gewebe entsprechenden Endothelschicht des Weiteren eine durch die Implantierung vermittelte inadäquate Proliferation von glatten Muskelzellen und/oder überschießende Bildung und Sekretion von Bestandteilen der extrazellulären Matrix reduzieren. Im Falle von koronaren Stents als bevorzugte erfindungsgemäße Implantate bedeutet das, dass das Risiko von Restenosen und/oder Spätthrombosen vermindert werden kann.

Erfindungsgemäß zu verwendende Aptamere haben den Vorteil, dass sie insbesondere aufgrund ihrer dreidimensionalen Struktur zum einen selektiver und/oder bindungsstärker als die üblicherweise verwendeten (c)RGD-Peptide an den αᵥβ₃-Integrin-Rezeptor binden können und somit das Risiko unspezifischer Integrinaktivierungen reduzieren. Andererseits sind erfindungsgemäß zu verwendende Aptamere im Vergleich zu (c)RGD-Peptiden sowohl in Bezug auf Sterilisierungsbehandlungen bei der Implantatherstellung als auch bezüglich physiologischer Abbauprozessen stabiler. Die erfindungsgemäße Verwendung von Aptameren kann im Vergleich zur Verwendung von freizusetzenden Wirkstoffen aus dem Stand der Technik zur Reduktion von unerwünschten Nebenwirkungen, wie Entzündungen im Gewebsbereich der Stentimplantation sowie Spätthrombosen, führen.

Im Sinne der vorliegenden Erfindung bedeutet "Aptamer mit aktivierender Wirkung gegenüber dem αᵥβ₃-Integrin-Rezeptor", dass einzelsträngige Oligo- bzw. Polynukleotide, vorzugsweise einzelsträngige Ribonukleinsäure (RNA), Desoxyribonukleinsäure (DNA) und/oder chemisch modifizierte Nukleinsäuren verwendet werden, die aufgrund ihrer dreidimensionalen Struktur so an den αᵥβ₃-Integrin-Rezeptor auf Endothelzellen binden, dass dieser von der inaktiven in die aktive Konformation übergeht und die entsprechende Signaltransduktion (so genanntes *"outside-in-signaling"*), die zur Endothelzelladhäsion an das Implantat führt, in Gang setzt.

Erfindungsgemäß geeignete Aptamere können mittels üblicher Verfahren, insbesondere des sogenannten "SELEX-Verfahrens" (systematic evolution of ligands by exponential enrichment) hergestellt werden. Das SELEX-Verfahren basiert auf zwei verschiedenen Verfahren zur in vitro Selektion von Nukleinsäuren, die im Jahr 1990 von Tuerk & Gold (Tuerk, C., Gold, L. (1990), "Systematic evolution of ligands by exponential enrichment: RNA ligands to bacteriophage T4 DNA polymerase", Science 249(4968):505-10) und Ellington & Szostak (Ellington, A.D, Szostak, J.W. (1990), "In vitro selection of RNA molecules that bind specific ligands", Nature 346(6287):818-22) publiziert wurden.

Das SELEX-Verfahren von Tuerk & Gold beginnt mit der Produktion von randomisierten, 10¹⁴ - 10¹⁵ verschiedenen RNA-Sequenzen, welche sich aufgrund ihrer variierenden Primärsequenzen unterschiedliche falten. Diese Bibliothek erhält man, indem man einen Bereich eines strukturell bekannten RNA-Oligonukleotids geringfügig verändert. Die Primer-Sequenzen am 3'- und 5'-Ende bleiben dabei immer gleich. Anschließend wird diese Bibliothek mit dem Zielprotein inkubiert und darauf folgend die gebundenen RNAs mittels Reverse Transkriptase Polymerasekettenreaktion (RT-PCR) amplifiziert. Anschließend wird durch eine in vitro Transkription eine neue Bibliothek gebildet, die mit RNA, welche an das Zielprotein bindet, angereichert ist. Dieser Selektions- und Amplifikationsprozess wird anschließend mindestens 4-mal wiederholt bis die RNA-Liganden mit der größten Affinität für das Zielprotein isoliert worden sind. Danach werden die so gewonnenen Liganden kloniert und sequenziert. Das Verfahren basiert dementsprechend auf den Vorgängen der Evolution, Variation, Selektion und Replikation.

Das Verfahren von Ellington & Szostak beschreibt die Selektion von RNA-Oligonukleotiden über Säulen mittels Affinitätschromatographie. Die hergestellte RNA-Bibliothek bestand aus 10¹³ verschiedenen RNA-Molekülen. Die Amplifikation der gewonnenen RNA-Liganden wurde ebenfalls durch eine PCR ermöglicht. Nach mindestens 5 Runden wurden die selektionierten Oligonukleotide kloniert und sequenziert. Ellington & Szostak verwendeten als Erste den Begriff "Aptamere".

Diese grundlegenden SELEX-Verfahren wurden bis heute immer weiter optimiert und sind im Stand der Technik hinreichend beschrieben.

Erfindungsgemäß zu verwendende Aptamere mit aktivierender Wirkung auf den αᵥβ₃-Integrin-Rezeptor können beispielsweise auch dadurch bereitgestellt werden, dass sie Oligonukleotide von Aptamere umfassen oder daraus bestehen, die zwar an den αᵥβ₃-Integrin-Rezeptor binden, aber eine Endothelzelladhäsion inhibieren, und ein oder mehrere der Phosphatgruppen des Zucker-Phosphatrückgrats zu ein oder mehreren, gleichen oder verschiedenen Thiophosphat- und/oder Methylphosphat-Gruppen, bevorzugt Thiophosphat, Dithiophosphat und Methylphosphate, derivatisiert sind. Nachfolgend wird beispielhaft in Formel 1 ein Ausschnitt einer 5'-3'Thiophosphat-Bindung zwischen zwei Nukleinsäuren (B steht für Nukleinbase) dargestellt:

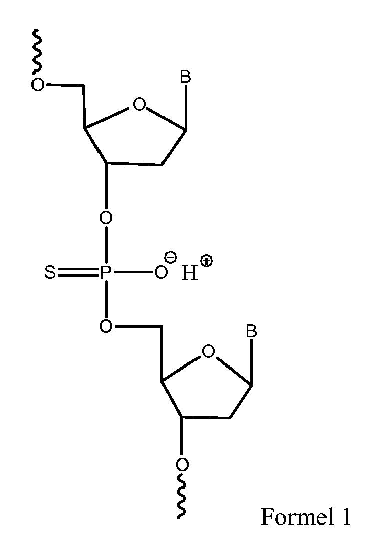

Thiophosphatgruppenderivatisierungen können wie folgt durchgeführt werden:
Eine Thiophosphatgruppe kann sowohl durch eine Wirkung von elementarem Schwefel in CS₂ auf Wasserstoffphosphonat als auch durch eine Sulfurierung von Phosphittriesetern mit einer Tetraethylthiuramdisulfide (TETD) oder einer 3H,1,2-Bensodithiol-3-one,1,1-dioxide (BDTD) Lösung hergestellt werden (http://www.sigmaaldrich.com/lifescience/custom-oligos/custom-dna/learning-center/phosphorothioates.html). Letzteres Verfahren umgeht das Problem der Unlöslichkeit des Schwefels in den meisten organischen Lösungsmitteln und der Toxizität des CS₂. Zudem erzielen die TETD- und BDTD-Verfahren Thiophosphate mit höherer Reinheit.

Thiophosphat- oder Methylphosphat-Derivatisierungen sind zwar im Stand der Technik dafür bekannt, dass sie Nukleinsäuren stabiler gegenüber RNAse-Abbau machen, jedoch hat sich erfindungsgemäß überraschend gezeigt, dass durch die entsprechenden Derivatisierungen nicht nur Nukleinsäuren erhalten werden können, die stabiler gegenüber RNAse-Spaltungen sind, sondern erfindungsgemäß derivatisierte Aptamere auch eine aktivierende Wirkung gegenüber dem αᵥβ₃-Integrin-Rezeptor aufweisen und so eine Endothelzelladhäsion vermitteln.

Aptamere, die zwar an den αᵥβ₃-Integrin-Rezeptor binden, aber eine Endothelzelladhäsion inhibieren, sind aus dem Stand der Technik bekannt und werden beispielsweise in folgenden Veröffentlichungen beschrieben: "Aptamer selection for the inhibition of cell adhesion with fibronectin as target", Ogawa, A. et al, Bioorganic & Medicinal Chemistry Letters 14 (2004) 4001 - 4004; "Targeted inhibition of aphavbeta3 integrin with an RNA aptamer impairs endothelial cell growth and survival", Mi, J. et al, Biochem Biophys Res Commun 2005, 338:956-963; ... (umfassend SEQ ID 1).

In einer bevorzugten erfindungsgemäßen Ausgestaltung umfasst oder besteht ein erfindungsgemäß zu verwendendes Aptamer mindestens zu 50%, vorzugsweise 60 bis 100% aus der Nukleinsäure eines Aptamers, das an den αᵥβ₃-Integrin-Rezeptor bindet, aber eine Endothelzelladhäsion inhibiert, wobei vorzugsweise zwischen 5 und 80%, weiter bevorzugt 10 bis 40% der Phosphatgruppen - wie oben beschrieben - erfindungsgemäß zu Thiophosphat- und/oder Methylphosphat-Gruppen derivatisiert sind und die Derivatisierungen vorwiegend am 5'- oder am 3'-Ende oder statistisch verteilt vorliegen können.

In einer weiteren bevorzugten erfindungsgemäßen Ausgestaltung umfasst oder besteht ein erfindungsgemäß zu verwendendes Aptamer aus SEQ ID 1, wobei zwischen 1 und 80, vorzugsweise 3 bis 20, weiter bevorzugt 5 bis 12 Phosphatgruppen zu Thiophosphat- und/oder Methylphosphat-Gruppen derivatisiert sind.

Zusätzlich können erfindungsgemäß geeignete Aptamere mittels geeigneter Verfahren so modifiziert, dass sie insbesondere unter physiologischen Bedingungen geschützt sind (reduzierter Abbau durch Nukleasen) und dennoch nicht ihre Wirksamkeit verlieren. Entsprechende Schutzverfahren sind im Stand der Technik hinreichend beschrieben und umfassen beispielsweise die Spiegelmer^{®}-Technologie als auch die LNA-Technologie (locked nucleic acids). Bei der Spiegelmer^{®}-Technologie können die positiven Eigenschaften der Aptamere auf spiegelbildliche Nukleinsäuren übertragen werden, die aus L-Nukleotiden bestehen und daher von Nukleinsäure-abbauenden Enzymen, z.B. ubiquitäre RNAsen, nicht erkannt werden. Diese Spiegelmere^{®} beweisen auch im Blutserum eine hohe Stabilität. Neben den Spiegelmeren^{®} ist es aber auch möglich selektierte, funktionelle Nukleinsäuren durch Modifikation des Zucker-Phosphat-Rückgrates gegen Nukleaseabbau zu stabilisieren. Dabei können beispielsweise Methylierungen und Fluorierungen durchgeführt oder auch so genannte Locked Nucleic Acids (LNA) in die Aptamere zur Stabilisierung eingebaut werden.

Implantate bzw. Implantatgrundkörper im Sinne der vorliegenden Erfindung können alle medizinischen, plastischen und/oder funktionellen Implantate bzw. Implantatgrundkörper darstellen und werden beispielsweise ausgewählt aus der Gruppe bestehend aus Herzschrittmacher; Hirnschrittmacher; Herzimplantate; Schrittmacherelektroden; Defibrillationselektroden; Cochleaimplantate; Implantate für Zahnmedizin; Endoprothesen, bevorzugt für Kniegelenke und/Hüftgelenke; Depotimplantate, die dazu dienen, ein Depot eines Wirkstoffs zu bilden; degradierbare oder dauerhafte, koronare oder periphere Stents; degradierbare oder dauerhafte Stents für andere Hohlräume, vorzugsweise die Speiseröhre, den Gallengang, die Harnröhre, die Prostata oder die Luftröhre; und local drug delivery (LDD)-Implantate, die vorzugsweise endovaskulär im Blut oder anderen Hohlräumen implantiert werden.

Die vorliegende Erfindung ist insbesondere auch für Implantate im Knochenbereich gut geeignet, da der αᵥβ₃-Integrin-Rezeptor auch auf reifen, knochenresorbierenden Osteoklasten ausgebildet wird und so bei der Knochenresorption einen wesentlichen Anteil hat.

In einer bevorzugten Ausgestaltung der vorliegenden Erfindung werden erfindungsgemäße Implantate bzw. Implantatgrundkörper ausgewählt aus der Gruppe bestehend aus Herzschrittmacher; Herzimplantate; Schrittmacherelektroden; Defibrillationselektroden; Shunts; degradierbare oder dauerhafte, koronare oder periphere Stents; Neurostents und local drug delivery (LDD)-Implantate, die vorzugsweise endovaskulär im Blut oder anderen Hohlräumen implantiert werden.

In einer besonders bevorzugten Ausgestaltung der vorliegenden Erfindung werden erfindungsgemäße Implantate bzw. Implantatgrundkörper ausgewählt aus der Gruppe der dauerhaften oder degradierbaren koronaren Stents (Koronarstents), wobei das Stentgrundkörpermaterial Metall und/oder Polymer, vorzugsweise Metall umfassen kann.

Die ursprünglichen mechanischen Funktionen eines Koronarstents, beispielsweise die Dilatierbarkeit, der geringe Recoil, die Stabilität über eine gewünschte Zeitdauer (im Falle degradierbarer Stents, beispielsweise bestehend aus Magnesium und seinen Legierungen) sowie die Flexibilität sollen in erfindungsgemäßen Stents als erfindungsgemäße Implantate üblicherweise beibehalten werden.

Im Folgenden werden erfindungsgemäß üblicherweise zu verwendende Implantat(grundkörper)-Materialien, bevorzugt Stentgrundkörper-Materialien sowie bevorzugte Ausgestaltungen hiervon beschrieben, wobei Metall gegenüber Polymer bevorzugt ist, da insbesondere Abbauprodukte von degradierbaren Polymerimplantaten selbst ein erhöhtes Risiko für Entzündungen und Thrombosen aufweisen:

### Degradierbarer Implantatgrundkörper, vorzugsweise degradierbarer Stentgrundkörper:

Im Sinne der vorliegenden Erfindung bedeutet "degradierbarer Implantat(grundkörper)", bevorzugt "degradierbarer Stent(grundkörper)" (im Folgenden auch synonym mit "Grundkörper" bezeichnet), dass der Grundkörper in physiologischer Umgebung, insbesondere im Gefäßsystem eines menschlichen oder tierischen Organismus degradiert, d. h., so abgebaut wird, dass der Stent seine Integrität verliert. Vorzugsweise degradieren erfindungsgemäß degradierbare Grundkörper erst dann, wenn die Funktionen des Implantats nicht mehr physiologisch sinnvoll bzw. notwendig sind. Bei degradierbaren Stents bedeutet das, dass der Stent vorzugsweise erst dann degradiert oder seine Integrität verliert, wenn das traumatisierte Gewebe des Gefäßes verheilt ist und der Stent somit nicht länger im Gefäßlumen verbleiben muss.

### Metallischer Grundkörper:

In einer bevorzugten erfindungsgemäßen Ausgestaltung umfasst oder besteht der degradierbare Werkstoff bevorzugt aus einem metallischen Werkstoff, der eine biokorrodierbare Legierung darstellt, wobei die Hauptkomponente der Legierung ausgewählt wird aus der Gruppe Magnesium, Eisen, Zink und/oder Wolfram; insbesondere wird für einen degradierbaren metallischen Werkstoff eine Magnesiumlegierung bevorzugt.

Die Legierung, insbesondere umfassend Magnesium, Eisen, Zink und/oder Wolfram, ist so in ihrer Zusammensetzung zu wählen, dass sie biokorrodierbar ist. Als biokorrodierbar im Sinne der vorliegenden Erfindung werden Legierungen bezeichnet, bei denen in physiologischer Umgebung ein Abbau (Degradierung) stattfindet, der letztendlich dazu führt, dass der gesamte Stent oder der aus dem Werkstoff gebildete Teil des Stents seine mechanische Integrität verliert. Unter Legierung wird vorliegend ein metallisches Gefüge verstanden, dessen Hauptkomponente Magnesium, Eisen, Zink und/oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, weiter bevorzugt mehr als 70 Gew.%. Eine Magnesium-Legierung ist hiervon besonders bevorzugt.

Ist der erfindungsgemäße Werkstoff eine Magnesium-Legierung, so enthält diese vorzugsweise Yttrium und weitere Seltenerdmetalle, da sich eine derartige Legierung aufgrund ihrer physiko-chemischen Eigenschaften und ihrer hohen Biokompatibilität, insbesondere auch ihrer Abbauprodukte, auszeichnet.

Besonders bevorzugt werden Magnesium-Legierungen der WE-Reihe, insbesondere WE43 sowie Magnesium-Legierungen der Zusammensetzung Seltenerdmetalle 5,5 bis 0,9 Gew.%, davon Yttrium 0,0 bis 5,5 Gew.% und Rest < 1 Gew.%, wobei der Rest Zirkonium und/oder Silicium enthalten kann und wobei in der Legierung Magnesium den auf 100 Gew.% fehlenden Anteil einnimmt, eingesetzt. Diese erfindungsgemäßen Magnesium-Legierungen bestätigen bereits experimentell und in ersten klinischen Versuchen ihre besondere Eignung, d. h. sie zeigen eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden in der vorliegenden Anmeldung Scandium (21), Yttrium (39), Lanthan (57) und die vierzehn an Lanthan (57) folgenden Elemente, nämlich Cer (58), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

### Polymer-Grundkörper:

Erfindungsgemäße Implantatgrundkörper, bevorzugt Stentgrundkörper, können gemäß einer weiteren alternativen erfindungsgemäßen Ausgestaltung degradierbares Polymer umfassen oder daraus bestehen, vorzugsweise ausgewählt aus der Gruppe bestehend aus Polydioxanon; Polyglycolid; Polycaprolacton; Polyhydroxyvaleriansäure; Polyhydroxybuttersäure; Polylactide, vorzugsweise Poly(L-lactid), Poly(D-lactid), Poly(D,L-lactid) und Blends sowie Copoylmere, vorzugsweise Poly(L-lactid-co-glycolid), Poly(D,L-lactid-co-glycolid), Poly(L-lactid-co-D-L-lactid), Poly(L-lactid-co-trimethylencarbonat) und Triblockcopolymere; Polysaccharide, vorzugsweise Chitosan, Levan, Hyaluronsäure, Heparin, Dextran und Cellulose.

### Dauerhafter Implantatgrundkörper, vorzugsweise dauerhafter Stentgrundkörper:

Im Gegensatz zum degradierbaren Grundkörper wird der "dauerhafte Implantatgrundkörper", vorzugsweise der "dauerhafte Stentgrundkörper" in physiologischer Umgebung im menschlichen oder tierischen Organismus im Wesentlichen nicht abgebaut, so dass er seine Integrität nicht verliert.

In einer weiteren erfindungsgemäßen bevorzugten Ausgestaltung umfasst oder besteht der Grundkörper eines dauerhaften Implantats, vorzugsweise eines dauerhaften Stents, aus einem Formgedächtnis-Material, vorzugsweise aus einem oder mehreren Materialien ausgewählt aus der Gruppe der Nickel-Titan-Legierungen und Kupfer-Zink-AluminiumLegierungen, weiter bevorzugt Nitinol.

In einer besonders bevorzugten erfindungsgemäßen Ausgestaltung umfasst oder besteht der Grundkörper eines dauerhaften Implantats, vorzugsweise eines dauerhaften Stents aus Edelstahl, vorzugsweise aus Cr-Ni-Fe-Stahl, hier bevorzugt die Legierung 316L, oder einem Co-Cr-Stahl.

In einer weiteren erfindungsgemäßen Ausgestaltung kann der Implantat-, vorzugsweise Stentgrundkörper zusätzlich Kunststoff, vorzugsweise Polyetherurethan, und/oder Keramik und/oder weitere Polymerbeschichtungen umfassen.

Werden im Sinne der vorliegenden Erfindung endovaskulär implantierbare Stents, vorzugsweise Koronarstents als implantierbare Grundkörper verwendet, so können alle üblichen Stentgeometrien für die vorliegende Erfindung verwendet werden. Insbesondere bevorzugt sind Stentgeometrien, die insbesondere in US 6,896,695, US 2006/241742, US 5,968,083 (Tenax), EP 1 430 854 (Helix-Design), US 6,197, 047 und EP 0 884 985 beschrieben werden.

Im Sinne der vorliegenden Erfindung bedeutet "Beschichtung mit einem oder mehreren, gleichen oder verschiedenen Aptameren", dass ein oder mehrere, gleiche oder verschiedene erfindungsgemäß zu verwendende Aptamere entweder auf der ganzen oder einem Teil der Oberfläche des Implantatgrundkörpers oder in Kavität(en) (beispielsweise im Falle von Stents als bevorzugten erfindungsgemäßen Implantaten) vorliegen können.

Sofern ein erfindungsgemäßes Implantat in einen Hohlraum des menschlichen oder tierischen Körpers, insbesondere in ein Blutgefäß implantiert wird und einerseits eine Oberfläche aufweist, die mit dem Gewebe in Kontakt steht (abluminale Oberfläche), als auch andererseits eine Oberfläche aufweist, die mit dem Inneren des Hohlraums, vorzugsweise des Blutgefäßes in Kontakt steht (luminale Oberfläche), dann liegt die erfindungsgemäße Beschichtung vorzugsweise auf (einem Teil) der abluminalen Oberfläche des erfindungsgemäßen Implantats vor. Durch diese Anordnung kann das Risiko von unerwünschten Nebenwirkungen, insbesondere durch Aktivierung von Integrin-Rezeptoren auf Zellen im Hohlraum, vorzugsweise Blutstrom, beispielsweise auf Thrombozyten, reduziert werden.

In einer bevorzugten Ausgestaltung ist ein erfindungsgemäßes Implantat dadurch gekennzeichnet, dass die Aptamere unabhängig voneinander unter physiologischen Bedingungen aus der Beschichtung (i) freigesetzt oder (ii) im Wesentlichen nicht freigesetzt werden können.

### (i) Freisetzung eines Aptamers aus der Beschichtung

Im Sinne der vorliegenden Anmeldung bedeutet, (i) Freisetzung eines Aptamers aus der Beschichtung, dass das Aptamer in einer geeigneten Art und Weise auf dem Implantatgrundkörper beschichtet ist, dass durch eine lokale Freisetzung aus der Beschichtung das Aptamer αᵥβ₃-Integrin-Rezeptoren im lokalen Bereich des Implantats, vorzugsweise an Endothelzellen, aktivieren kann.

Im Folgenden werden Beispiele genannt für erfindungsgemäß geeignete Beschichtungen, die geeignet sind, (i) Aptamere unter physiologischen Bedingungen freizusetzen:
- Beschichtung der ganzen oder eines Teils der Oberfläche des Implantatgrundkörpers mit einer Polymer-Aptamer-Mischung, wobei das Polymer natürlichen oder synthetischen Ursprungs und/oder degradierbar oder nicht degradierbar sein kann.
   Beispielsweise kann ein Implantatgrundkörper mit einem Komposit umfassend Aptamere und einem oder mehreren, vorzugsweise einem hydrophoben, biodegradierbaren Polymer beschichtet werden. Geeignete Komposite werden beispielsweise in DE 10 2006 038 240.4 (BIOTRONIK VI Patent AG) beschrieben und der Offenbarungsgehalt wird in diesem Zusammenhang vollständig in die vorliegende Anmeldung inkorporiert. Dementsprechend können geeignete Komposite beispielsweise wie folgt hergestellt werden:
   (i) Herstellen einer wässrigen Lösung aus einem Polyethylenglycol (PEG) mit einem mittleren Molekulargewicht im Bereich von 600 bis 30.000 g/mol, vorzugsweise 12.000 bis 30.000 g/mol,
   (ii) Zugabe einer wässrigen, gegebenenfalls gepufferten Lösung von Aptameren, vorzugsweise 0,1 bis 7 mg/ml Aptamer, weiter bevorzugt 5 mg/ml Aptamer, zur wässrigen Lösung aus Schritt (i), wobei in der resultierenden wässrigen Lösung PEG vorzugsweise im 1,2 bis 100 fachen gravimetrischen Überschuss vorliegt,
   (iii) Einfrieren (vorzugsweise bei -25°C) der wässrigen Lösung aus Schritt (ii) und anschließendes Gefriertrocknen (vorzugsweise bei vermindertem Druck, bspw.
      3x10⁻⁶, über vorzugsweise 24 Stunden, um sicherzustellen, dass das Wasser komplett entfernt wurde) der gefrorenen Lösung unter Erhalt eines gefriergetrockneten Substrats (watteartige Konsistenz),
   (iv) Bereitstellen einer Lösung eines hydrophoben, biodegradierbaren Polymers, vorzugsweise Polymilchsäure, weiter bevorzugt Poly-L-Milchsäure, in einem organischen Lösungsmittel, vorzugsweise Aceton, Chloroform, Ethanol, Ethylacetat, Acetylaceton, Hexyfluoroisopropanol, Tetryhydrofuran (THF) und Dichlormethan, besonders bevorzugt Chloroform,
   (v) Zugabe des nach Schritt (iii) erhaltenen Substrats zur Lösung des Polymers aus Schritt (iv) und
   (vi) Entfernen des organischen Lösungsmittels, vorzugsweise unter vermindertem Druck, unter Erhalt des Komposits.
- Beschichtung von Kavität(en) des Implantatgrundkörpers mit einer geeigneten Aptamer-Mischung, vorzugsweise einer Hydrogel-Aptamer-Mischung, bei der das Aptamer vorzugsweise in Mengen zwischen 2 bis 50 Gew.%, weiter bevorzugt zwischen 10 bis 25 Gew.% vorliegt. Bei dieser Ausgestaltung werden die Aptamere vorzugsweise durch Quellung und / oder Diffusion aus den Kavitäten freigesetzt.
- Beschichtung der ganzen oder eines Teils der Oberfläche des Implantatgrundkörpers mit Nanopartikeln, die Polymer und Aptamer umfassen, wobei das Polymer natürlichen oder synthetischen Ursprungs und/oder degradierbar oder nicht degradierbar sein kann, vorzugsweise positiv geladenes Polymer, beispielsweise hydrophile Polymere wie Polyethylenimin, Polylysin und Chitosan oder hydrophobe Polymere wie Polylactide, vorzugsweise Poly(L-lactid), Poly(D-lactid), Poly(D,L-lactid) und Blends sowie Copoylmere, vorzugsweise Poly(L-lactid-co-glycolid), Poly(D,L-lactid-co-glycolid), Poly(L-lactid-co-D-L-lactid) und Poly(L-lactid-co-trimethylencarbonat).

Beispiel für Herstellung von Nanopartikel umfassend Polyethylenimin und/oder Polylysin und Aptamer: Das positiv geladene, hydrophile Polymer gibt mit der negativ geladenen Nukleinsäure des Aptamers in Wasser unlösliche Niederschläge, die bei entsprechender Rührgeschwindigkeit (beispielsweise 300 UPM) und Zusatz von Detergentien, wie beispielsweise 0,1% Alkylphenylpolyethylenglykol (Triton^{®}X100) als Nanopartikel ausfallen.

Beispiel für Herstellung von Nanopartikel umfassend Chitosan und Aptamer: 5 mg Chitosan werden in 1 ml einer 0,5 Gew.% Essigsäurelösung gegeben. Zu dieser Lösung werden 1 ml Aptamerlösung (5,5 mg/ml) gegeben und dabei stark gerührt. Der feine Niederschlag wird vorzugsweise in phosphatgepufferte Salzlösung (PBS Puffer) 50 mM aufgenommen und weiterverarbeitet.

Für die Herstellung von Nanopartikels umfassend hydrophobe Polymere und Aptamer können Emulsionsverfahren (Wasser/Öl/Wasser) oder (Wasser/Öl/Öl) verwendet werden, um die rein wasserlöslichen Aptamere in ein in Wasser unlösliches Polymer zu überführen. Entsprechende Herstellverfahren können beispielsweise "Biomaterials for Delivery and Targeting of Proteins and Nucleic acids": ISBN0-8493-2334-7 (2005)CRC press; Edited by Ram I. Mahato entnommen werden.

### (ii) Beschichtung aus der Aptamere im Wesentlichen nicht freigesetzt werden

Im Sinne der vorliegenden Anmeldung bedeutet (ii) "Beschichtung aus der Aptamere im Wesentlichen nicht freigesetzt werden", dass das Aptamer direkt oder über eine oder mehrere, gleiche oder verschiedene Ankergruppen mit der gegebenenfalls funktionalisierten Oberfläche des Implantatgrundkörpers so verbunden ist, dass das Aptamer unter physiologischen Bedingungen nach Implantation über einen unbestimmten Zeitraum im Wesentlichen nicht freigesetzt wird. In einer entsprechenden Beschichtung aktiviert das Aptamer lokal im Bereich der Implantation αᵥβ₃-Integrin-Rezeptoren, vorzugsweise auf angrenzenden Endothelzellen. Diese erfindungsgemäße Ausgestaltung hat den Vorteil, dass auf Grund der Abwesenheit von Polymer in der Beschichtung insbesondere keine Polymerabbauprodukte vorliegen und somit das Risiko von entsprechend induzierten Entzündungen bzw. Thrombosen reduzieren. Des Weiteren kann die erfindungsgemäße Verwendung von Aptameren, die im Wesentlichen nicht aus der Beschichtung freigesetzt werden, aufgrund des Fehlens einer freizusetzenden Substanz und einer Polymerbeschichtung zu einem vereinfachten Zulassungsverfahren führen.

Erfindungsgemäß geeignete Ankergruppen umfassen 1,4-Phenylendiisothiocyanat, N-succinimidyl3-(2-pyridyldithio)propionat und/oder ein lineares oder sternförmiges PEG-Block-Coplymer (siehe hierzu auch WO 2004/055153), oder werden ausgewählt aus der Gruppe an Verbindungen der allgemeinen Formel (I) (siehe hierzu auch DE 10 2008 040 573.6, BIOTRONIK VI Patent AG) (R²O)₂(O)P-L-(CH₂)x-M-R¹(I) wobei
- R¹: für -COOH, -OH, -SH, -NH₂, Benzophenon oder Benzophenon-Derivate steht,
- R²: für Wasserstoff, -CH₂CH₃ oder -CH₃ steht,
- L: für eine Einfach-Bindung oder -O- steht,
- M: für eine Einfach-Bindung oder -(CH₂-CH₂-O)y steht,
- x: für eine Zahl ausgewählt aus der Gruppe bestehend aus 1 bis 25 steht und
- y: für eine Zahl ausgewählt aus der Gruppe bestehend aus 1 bis 25 steht.

Im Sinne der vorliegenden Erfindung sind unter dem Begriff "Benzophenon-Derivat" alle üblichen Derivate zu fassen. Bevorzugt werden die Derivate ausgewählt aus der Gruppe der Oxybenzophenone.

Demzufolge fallen unter Verbindungen der allgemeinen Formel (I)
Phosphonsäuren der allgemeinen Formel (II)
(HO)₂(O)P-O(CH₂)x-M-R¹ (II) und
deren Ester der allgemeinen Formel (III)
(R²O₂(O)P-CH₂)_{X}-M-R¹ (III) sowie
Verbindungen der phosphorigen Säure der allgemeinen Formel (IV)
(HO)₂(O)P-O-(CH₂)x-M-R¹ (IV)
und/oder deren Ester der allgemeinen Formel (V)
(R²O)₂(O)P-O(CH₂)x-M-R¹ (V),
wobei die (bevorzugten) Reste
R¹, R², M, und x gemäß der allgemeinen Formel (I) definiert sind und die bevorzugten Reste unabhängig voneinander vorhanden sein können.

Besonders bevorzugt werden Verbindungen der allgemeinen Formel (I) ausgewählt aus der Gruppe bestehend aus Hydroxyundecylphosphonsäure, 3-(4-Oxybenzophenon)propylphosphonsäure und Carboxydodecylphosphonsäure.

Verbindungen der allgemeinen Formeln (I), (II), (III), (IV) und (V), wobei M für einen Oligoethylenglykol-Linker -((CH₂)CH₂O)Y- steht, sind insbesondere bevorzugt, da hierdurch weiterhin eine unspezifische Proteinanlagerung an diese Ankergruppen im Gegensatz zu Ankergruppen, in denen M für eine Einfach-Bindung steht, vermindert wird.

Verfahren zur Immobilisierung von Aptameren werden alternativ auch in "Immobilisierung von Oligonukleotiden an aminofunktionalisierte Silizium-Wafer" (U. Hacker, Chem. Diss., Hamburg; Einsatz von 1,4-Phenylendiisothiocyanat) und in "Miniaturisierte Affinitätsanalytik - Ortsaufgelöste Oberflächenmodifikationen, Assays und Detektion" (I. Stemmler, Chem. Diss., Tübingen, 1999) beschrieben.

Gemäß einer weiteren erfindungsgemäßen Ausgestaltung kann ein erfindungsgemäßes Implantat zusätzlich ein oder mehrere Wirkstoffe umfassen. Ein Wirkstoff im Sinne dieser Erfindung ist eine Substanz oder eine Verbindung, die im menschlichen oder tierischen Körper eine biologische Reaktion hervorruft. In diesem Sinne kann Wirkstoff auch synonym zu Arzneistoff und/oder Pharmakon verwendet werden. Im Sinne der vorliegenden Erfindung ist der erfindungsgemäße Stent mit ein oder mehreren Wirkstoffen in einer Konzentration beschichtet, die ausreicht, die gewünschten physiologischen Reaktionen hervorzurufen.

Erfindungsgemäß zu verwendende Wirkstoffe werden bevorzugt ausgewählt aus der Gruppe bestehend aus: Antiphlogistika, vorzugsweise Dexamethason, Methylprednisolon und Diclophenac; Cytostatika, vorzugsweise Paclitaxel, Colchicin, Actinomycin D und Methotrexat; Immunsuppressiva, vorzugsweise Limus-Verbindungen, weiter bevorzugt Sirolimus (Rapamycin), Zotarolimus (Abt-578), Tacrolimus (FK-506), Everolimus, Biolimus, insbesondere Biolimus A9 und Pimecrolimus, Cyclosporin A und Mycophenolsäure; Thrombozytenaggregationshemmer, vorzugsweise Abciximab und Iloprost; Statine, vorzugsweise Simvastatin, Mevastatin, Atorvastatin, Lovastatin, Pitavastatin und Fluvastatin; und Estrogene, vorzugsweise 17b-Estradiol, Daizein und Genistein; Lipidregulatoren, vorzugsweise Fibrate; Immunsuppressiva; Vasodilatatoren, vorzugsweise Satane; Calciumkanalblocker; Calcineurininhibitoren, vorzugsweise Tacrolimus; Antiinflammatorika, vorzugsweise Imidazole; Antiallergika; Oligonucleotide, vorzugsweise Decoy-Oligodesoxynukleotid (dODN); Endothelbildner, vorzugsweise Fibrin; Steroide; Proteine/Peptide; Proliferationshemmer; Analgetika und Antirheumatika.

Besonders bevorzugt werden erfindungsgemäß Paclitaxel und Limus-Verbindungen, weiter bevorzugt Sirolimus (Rapamycin), Zotarolimus (Abt-578), Tacrolimus (FK-506), Everolimus, Biolimus, insbesondere Biolimus A9 und Pimecrolimus, ganz besonders bevorzugt Rapamycin (Sirolimus) als der oder die weiteren Wirkstoffe verwendet.

Ein erfindungsgemäßer peripherer oder koronarer Stent wird vorzugsweise auf (einem Teil) der abluminalen Oberfläche, also der Oberfläche, die vorwiegend mit dem umliegenden Gewebe und nicht dem Gefäßlumen des Blutgefäßes nach Implantation in Kontakt steht, mit dem oder den Wirkstoffen beschichtet, da bei einer luminalen Beschichtung die Degradation des Stents, vorzugsweise eines biodegradierbaren Stents und besonders bevorzugt eines biodegradierbaren Metallstents wesentlich beeinträchtigt werden kann.

In einer weiteren bevorzugten Ausgestaltung kann ein mit Wirkstoff(en) beschichtetes erfindungsgemäßes Implantat gegebenenfalls eine oder mehrere weitere Beschichtungen auf (einem Teil) der Oberfläche des Implantatgrundkörpers, vorzugsweise auf der Oberfläche der wirkstoffhaltigen Schicht, als sogenannten "Topcoat" aufweisen, um die Abrasion der Wirkstoffbeschichtung bei Implantation zu verringern.

Für den Fall, dass ein oder mehrere unterschiedliche Polymere für die Wirkstoffbeschichtung und/oder den Topcoat verwendet werden, werden sie üblicherweise ausgewählt aus der Gruppe bestehend aus:
- nicht degradierbare Polymere: Polyethylen; Polyvinylchlorid; Polyacrylate; vorzugsweise Polyetyl- und Polymethylacrylate, Polymethylmetacrylat, Polymethyl-co-ethylacrylat und Ethylen/Ethylacrylat; Polytetrafluorethylen, vorzugsweise Ethylen/Chlortrifluorethylen Copolymere, Ethylen/Tretrafluorethylen Copolymere; Polyamide (PA), vorzugsweise Polyamidimid, PA-11, PA-12, PA-46 oder PA-66; Polyetherimid; Polyethersulfon; Poly(iso)butylen; Polyvinylchlorid; Polyvinylfluorid; Polyvinylalkohol; Polyurethan; Polybuthylenterephthalat; Silikone; Polyphosphazen; Polymerschäume, vorzugsweise Polymerschäume aus Carbonaten, Styrolen; Copolymere und/oder Blends der aufgezählten Polymerklassen, Polymere der Klasse der Thermoplaste sowie
- degradierbare Polymere: Polydioxanon; Polyglycolid; Polycaprolacton; Polylactide, vorzugsweise Poly-L-lactid, Poly D,L-lactid, und Copolymere sowie Blends hiervon, vorzugsweise Poly(L-lactid-co-glycolid), Poly(D,L-lactid-co-glycolid), Poly(L-lactid-co-D,L-lactid), Poly(L-lactid-co-trimethylen carbonat); Triblockcopolymere; Polysaccharide, vorzugsweise Chitosan, Levan, Hyaluronsäure, Heparin, Dextran, Cellulose; Polyhydroxyvalerat; Ethylvinylacetat; Polyethylenoxid; Polyphosphorylcholin; Fibrin; Albumin; Polyhydroxybuttersäure, vorzugsweise ataktische, isotaktische und/oder syndiotaktische Polyhydroxybuttersäure sowie deren Blends.

Besonders bevorzugte Polymere für die wirkstoffhaltige Schicht und/oder den Topcoat der vorliegenden Erfindung sind die oben beschriebenen degradierbaren Polymere, da durch den vollständigen Abbau des oder der Polymere kein körperfremder Bestandteil im Organismus verbleibt.

Die bevorzugten Ausgestaltungen des erfindungsgemäß verwendbaren Implantats, vorzugsweise des erfindungsgemäßen Stents können in allen denkbaren Varianten untereinander, aber auch mit den weiteren hierin offenbarten bevorzugten Ausgestaltungen kombiniert werden.

Gemäß der zweiten erfindungsgemäßen Ausgestaltung wird ein Verfahren zur Herstellung eines erfindungsgemäßen Implantates bereitgestellt, bei dem die Aptamere unabhängig voneinander aus der Beschichtung freigesetzt werden können.

Ein geeigneter Implantatgrundkörper, der in Schritt a) bereitgestellt wird, wird im Zusammenhang mit dem erfindungsgemäßen Implantat sowie seinen bevorzugten Ausgestaltungen beschrieben. Der Implantat-, bevorzugt Stentgrundkörper kann auch funktionalisiert oder derivatisiert sein, beispielsweise mit einer Parylenschicht, insbesondere einer aminofunktionalisierten Parylenschicht.

Gegebenenfalls kann der Implantat-, bevorzugt Stentgrundkörper mittels üblicher Verfahren gereinigt werden.

Ein geeignetes Beschichtungsmaterial umfassend ein oder mehrere, gleiche oder verschiedene Aptamere, die eine aktivierende Wirkung gegenüber dem αᵥβ₃-Integrin-Rezeptor aufweisen, wobei das Beschichtungsmaterial geeignet ist, das oder die Aptamere unter physiologischen Bedingungen aus der Beschichtung freizusetzen, wird ebenfalls im Zusammenhang mit dem erfindungsgemäßen Implantat sowie seinen bevorzugten Ausgestaltungen beschrieben. Gemäß einer bevorzugten Ausgestaltung wird ein Komposit aus Aptamer(en) und einem hydrophoben, biodegradierbaren Polymer, wie ebenfalls im Zusammenhang mit dem erfindungsgemäßen Implantat beschrieben, verwendet.

Die Beschichtung des erfindungsgemäßen Implantatgrundkörpers, vorzugsweise Stentgrundkörpers, mit dem Beschichtungsmaterial aus Schritt b) auf mindestens einem Teil der Oberfläche oder in Kavität(en) des Implantatgrundkörpers kann gemäß üblicher Verfahren bewerkstelligt werden. Insbesondere kann hierzu das Beschichtungsmaterial aus Schritt b) mittels eines Tauchverfahrens (Dippcoating), einer Sprühbeschichtung mittels Ein- bzw. Mehrstoffdüse, Rotationszerstäubung und Druckdüsen oder Sputtern auf die Oberfläche des Implantatgrundkörpers aufgetragen werden. Kavität(en) können üblicherweise selektiv durch Bestreichen oder Befüllen beschichtet werden.

Für den Fall, dass ein erfindungsgemäßes Implantat zusätzlich ein oder mehrere Wirkstoffe umfasst, können diese entweder im Beschichtungsmaterial aus Schritt b) umfasst sein oder der oder die Wirkstoffe können in einer separaten Schicht auf (einen Teil) der Oberfläche des Implantatgrundkörpers, vorzugsweise der Oberfläche des Implantatgrundkörpers, die nicht mit Aptamer beschichtet ist, mittels üblicher Verfahren aufgebracht werden. Insbesondere kann hierzu eine reine Wirkstoffschmelze, ein Wirkstoff-Lösungsmittelgemisch oder ein Wirkstoff-Polymergemisch beispielsweise mittels eines Tauchverfahren (Dippcoating), einer Sprühbeschichtung mittels Ein- bzw. Mehrstoffdüse, Rotationszerstäubung und Druckdüsen, Sputtern auf (einen Teil) der Oberfläche des Implantatgrundkörpers aufgetragen werden. Für den Fall, dass zusätzlich ein oder mehrere Topcoats auf dem erfindungsgemäßen Implantat vorliegen, können die vorstehenden Beschichtungsmethoden auch hierfür angewendet werden.

Für den Fall, dass vorwiegend die abluminale Oberfläche eines erfindungsgemäßen Stents mit Aptameren, ein oder mehreren Wirkstoffen und/oder einem Topcoat beschichtet werden soll, kann dies vorzugsweise dadurch bewerkstelligt werden, dass in den vorgenannten Verfahren der Implantatgrundkörper, vorzugsweise Stentgrundkörper auf beispielsweise einen Zylinder, Kanüle oder Dorn aufgesteckt wird, damit nur die abluminale Oberfläche des Stents mit einer Aptamer bzw. einer Wirkstoffschicht beschichtet wird. Alternativ könnte die abluminale Beschichtung mit mittels Walzenauftrag oder das selektive Auftragen durch Bestreichen oder Befüllen von Kavitäten vorgenommen werden.

Gegebenenfalls können einem oder mehreren Beschichtungsschritten ein üblicher Trocknungsschritt oder andere übliche physikalische oder chemische Nachbearbeitungsschritte, z. B. Vakuum- oder Plasmabehandlung, folgen, bevor das erfindungsgemäße Implantat, vorzugsweise Stent, weiter behandelt wird.

Gemäß der dritten erfindungsgemäßen Ausgestaltung wird ein Verfahren zur Herstellung eines erfindungsgemäßen Implantates bereitgestellt, bei dem das Aptamer (ii) im Wesentlichen nicht aus der Beschichtung freigesetzt wird.

Ein geeigneter Implantatgrundkörper, der in Schritt a) bereitgestellt wird, wird auch hier im Zusammenhang mit dem erfindungsgemäßen Implantat sowie seinen bevorzugten Ausgestaltungen beschrieben. Erfindungsgemäß ist dieser bevorzugt weder funktionalisiert noch derivatisiert.

Geeignete Ankergruppen, die in Schritt b) bereitgestellt werden, werden ebenfalls im Zusammenhang mit dem erfindungsgemäßen Implantat sowie seinen bevorzugten Ausgestaltungen beschrieben, wobei diese üblicherweise in einem Lösungsmittel, vorzugsweise ausgewählt aus der Gruppe umfassend Methanol, Ethanol, Aceton, Tetrahydrofuran (THF), Dimethylformamid (DMF), Chloroform, Dimethylsulfoxid (DMSO), Dichlormethan, gelöst, suspendiert oder emulgiert vorliegen.

In einer bevorzugten erfindungsgemäßen Ausgestaltung wird der in Schritt a) bereitgestellte Implantat-, vorzugsweise Stentgrundkörper mittels üblicher Verfahren so gereinigt, dass der Grundkörper so aktiviert wird, dass die in Schritt b) bereitgestellten Ankergruppen in Schritt c) bevorzugt gebunden werden. Bevorzugt wird der Implantat-, vorzugsweise Stentgrundkörper in einem Sauerstoffplasma und/oder durch Spülen mit Lösungsmittel, bevorzugt der Lösungsmittelreihe Dichlormethan, Aceton, Methanol und Millipore Wasser zu verstehen. Gegebenenfalls kann diesem Reinigungsschritt ein üblicher Trocknungsschritt folgen.

Unter dem erfindungsgemäßen Funktionalisieren des Implantat-, vorzugsweise Stentgrundkörpers in Schritt c) mit der oder den Ankergruppen aus Schritt b) ist üblicherweise das vollständige oder teilweise In-Kontakt-Bringen des (gereinigten) Implantat-, vorzugsweise Stentgrundkörpers mit einer oder mehreren Lösungen gleicher oder verschiedener Ankergruppen zu verstehen, wobei das oder die Lösungsmittel anschließend ganz oder teilweise entfernt, bevorzugt abgedampft werden. In einer bevorzugten Ausgestaltung wird das oder die Lösungsmittel innerhalb einer Stunde so entfernt, bevorzugt abgedampft, dass der Meniskus der Lösung über die Implantat-, vorzugsweise Stentoberfläche wandert. Unter "In-Kontakt-Bringen" ist erfindungsgemäß üblicherweise ein Besprühen des (gereinigten) Grundkörpers mit oder ein Eintauchen des (gereinigten) Grundkörpers in ein oder mehrere Lösungen gleicher oder unterschiedlicher Ankergruppen zu verstehen.

Anschließend kann gegebenenfalls der mit einer oder mehreren Ankergruppen funktionalisierte Implantat-, vorzugsweise Stentgrundkörper thermisch behandelt (getempert) werden, um die Anbindung der Ankergruppen an den Grundkörper zu erhöhen. Bevorzugt wird über einen Zeitraum von 1 bis 124 Stunden und/oder in einem Temperaturbereich von 60°C bis 120°C, weiter bevorzugt für 18 bis 74 Stunden bei 100°C bis 140°C getempert.

Gegebenenfalls wird der funktionalisierte Implantat-, vorzugsweise Stentgrundkörper - funktionalisiert oder nicht - anschließend mit Lösungsmittel gespült.

Gegebenenfalls wird anschließend der so vorbehandelte funktionalisierte Implantat-, vorzugsweise Stentgrundkörper teilweise oder vollständig in eine Carbonyldiimidazol-Lösung (CDI) vorzugsweise in trockenem Dioxan gelegt. Vorzugsweise wird der Grundkörper 15 Stunden in eine 0,3M Lösung von Carbonyldiimidazol in trockenem Dioxan gegeben. Gegebenfalls schließt eine Spülung mit trockenem Dioxan und/oder ein Trocknungsschritt an.

Geeignete erfindungsgemäß zu verwendende Aptamere, die im Schritt d) bereitgestellt werden, werden ebenfalls im Zusammenhang mit dem erfindungsgemäßen Implantat sowie seinen bevorzugten Ausgestaltungen beschrieben und werden üblicherweise als Lösung, Suspension oder als Emulsion bereitgestellt. In einer bevorzugten Ausgestaltung liegen die Aptamere in einer vorzugsweise aminosäurefreien und/oder RNAsefreien Puffer-Lösung, bevorzugt PBS-Puffer (phosphat buffered saline), MES-Puffer (2-Morpholinoethanesulfonic acid), Borat-Puffer etc., weiter bevorzugt PBS-Puffer vor.

Der erfindungsgemäß funktionalisierte Implantat-, vorzugsweise Stentgrundkörper aus Schritt c) wird in Schritt e) ganz oder teilweise mit der Lösung, Suspension oder Emulsion der Aptamere aus Schritt d) in Kontakt gebracht und gegebenenfalls gespült. Unter "In-Kontakt-Bringen" ist üblicherweise ein Besprühen des funktionalisierten Grundkörpers mit oder ein Eintauchen in Lösungen, Suspensionen oder Emulsionen gleicher oder verschiedener Aptamere zu verstehen. In einer bevorzugten Ausgestaltung schließt hieran ein Trocknungsschritt, vorzugsweise mit Stickstoff an.

Für den Fall, dass Verbindungen der Formel (I), (11), (III) oder (IV) mit Benzophenonankergruppen verwendet werden, folgt zur Ausbildung der Bindung der erfindungsgemäß zu verwendenden Aptamere an den Implantat-, vorzugsweise Stentgrundkörper, ein Belichtungsschritt, üblicherweise ab 260 nm, vorzugsweise bei 360 nm mit 100 mW/cm².

Zur Ausbildung der Bindung der Endothelzellen an Carbonyl-Ankergruppen der Verbindungen der Formel (I), (II), (III) oder (IV) folgt vorzugsweise zuerst eine Aktivierung der Carbonyl-Ankergruppe mit N,N-(3-Dimethylaminopropyl)-N'-ethyl-carbodiimid hydrochlorid (EDC) und/oder N-Hydroxysuccinimid (NHS) und daran anschließend erst die Kopplung mit dem erfindungsgemäß einzusetzenden Aptamer.

Für die erfindungsgemäß bereitgestellten Herstellverfahren können, sofern für einen Fachmann sinnvoll, die bevorzugten Ausgestaltungen der auch im Zusammenhang mit der ersten erfindungsgemäßen Ausgestaltung des Implantats beschriebenen Merkmale miteinander kombiniert werden.

Für die vierte erfindungsgemäße Ausgestaltung der Verwendung eines Aptamers mit aktivierender Wirkung gegenüber dem αᵥβ₃-Integrin-Rezeptor zur Vermittlung Endothelzelladhäsion an ein Implantat für einen menschlichen oder tierischen Körper können ebenfalls, sofern für einen Fachmann sinnvoll, die bevorzugten Ausgestaltungen der auch im Zusammenhang mit der ersten, zweiten oder dritten erfindungsgemäßen Ausgestaltung des Implantats und dessen Herstellverfahren beschriebenen Merkmale miteinander kombiniert werden.

Für die fünfte erfindungsgemäße Ausgestaltung der Verwendung eines oder mehrerer unterschiedlicher Aptamere mit aktivierender Wirkung gegenüber dem αᵥβ₃-Integrin-Rezeptor zur vollständigen oder teilweisen Beschichtung eines Implantatgrundkörpers für einen menschlichen oder tierischen Körper können ebenfalls, sofern für einen Fachmann sinnvoll, die bevorzugten Ausgestaltungen der auch im Zusammenhang mit der ersten, zweiten oder dritten erfindungsgemäßen Ausgestaltung des Implantats und dessen Herstellverfahren beschriebenen Merkmale miteinander kombiniert werden.

Für die sechste erfindungsgemäße Ausgestaltung der Verwendung eines oder mehrerer unterschiedlicher Aptamere mit aktivierender Wirkung gegenüber dem αᵥβ₃-Integrin-Rezeptor zur Herstellung eines erfindungsgemäßen Implantats, können ebenfalls, sofern für einen Fachmann sinnvoll, die bevorzugten Ausgestaltungen der auch im Zusammenhang mit der ersten, zweiten oder dritten erfindungsgemäßen Ausgestaltung des Implantats und dessen Herstellverfahren beschriebenen Merkmale miteinander kombiniert werden.

### Ausführungsbeispiele:

Die vorliegende Erfindung wird im Folgenden durch Ausführungsbeispiele beschrieben, die jedoch den Schutzumfang des erfindungsgemäßen Gegenstandes nicht limitieren.

### Beispiel 1: Aptamerbeschichtung für einen Stentgrundkörper aus welcher Aptamer freigesetzt werden kann

### Herstellung des Aptamerbeschichtungsmaterials:

Es wird bei Raumtemperatur Polyethylenglycol (PEG) mit 12.000 -30.000 g/mol Molmasse in Wasser, vorzugsweise destilliertes Wasser gelöst. Zu dieser Lösung wird ebenfalls bei Raumtemperatur eine wässrige oder gepufferte Lösung eines oder mehrerer Aptamere (5 mg/ml) mit aktivierender Wirkung gegenüber dem αᵥβ₃-Integrin-Rezeptor gegeben, so dass ein 1,2 bis 100facher gravimetrischer Überschuss des PEGs vorliegt. Nach Herstellung einer homogenen Lösung, wird die erhaltene Lösung abgekühlt, vorzugsweise auf -25°, die so erhaltene gefrorene Substanz mechanisch zerkleinert und anschließend vorzugsweise bei vermindertem Druck (3x10⁻⁶ Bar) über 24 Stunden gefriergetrocknet, um sicherzustellen, dass das Wasser im Wesentlichen vollständig entfernt wurde.

Es entsteht eine watteartige Substanz. Diese watteartige Substanz ist chloroformlöslich und wird zu einem in Chlorform gelösten hydrophoben, biodegradierbaren Polymer, vorzugsweise einem Polylactid, vorzugsweise Poly-L-lactid, Poly-D,L-lactid, und Copolymere sowie Blends hiervon, vorzugsweise Poly(L-lactid-co-glycolid), Poly(D,L-lactid-co-glycolid), Poly(L-lactid-co-D,L-lactid), Poly(L-lactid-co-trimethylen carbonat) etc. gegeben. Der Aptamer-PEG-Komplex liegt solubilisert (quasi gelöst) vor.

Die Oberfläche eines gegebenenfalls im Sauerstoffplasma (Gerät der Fa. Diener; Modell Tetra; 200 W; 0,2 mBar; 2-30 Min.) oder durch Spülen mit der Lösungsmittelreihe Dichlormethan, Aceton, Methanol und Millipore-Wasser gereinigter Stentgrundkörper wird ganz oder teilweise mit dem vorstehend hergestellten Aptamerbeschichtungsmaterial beschichtet.

### Beispiel 2: Aptamerbeschichtung für einen Stentgrundkörper aus welcher Aptamer im Wesentlichen nicht freigesetzt werden kann

Ein im Sauerstoffplasma (Gerät der Fa. Diener; Modell Tetra; 200 W; 0,2 mBar; 2-30 Min.) oder durch Spülen mit der Lösungsmittelreihe Dichlormethan, Aceton, Methanol und Millipore-Wasser gereinigter Stent wird im Folgenden weiterbehandelt:
Es wird eine 1 mM Lösung von Hydroxyundecylphosphonsäure in trockenem Tetrahydrofuran hergestellt. In diese wird der Stent gehängt und das Lösungsmittel wird innerhalb von einer Stunde abgedampft, wobei der Meniskus der Lösung über die Stentoberfläche abwandert.

Anschließend wird der Stent 18 h bei 120°C getempert und daraufhin im Lösungsmittel gespült. Der so vorbehandelte Stent wird 15 h in eine 0.3 M Lösung von Carbonyldiimidazol (CDI) in trockenem Dioxan gelegt. Anschließend wird er 2x 10 Minuten mit trockenem Dioxan gespült und im Stickstoffstrom getrocknet.

Auf die so behandelten Stents wird eine Lösung zu der oben beschriebenen erfindungsgemäß einzusetzenden Aptameren (ca. 50 µg/ml), in PBS-Puffer (vorzugsweise aminosäurefrei, RNAse-frei) gegeben und über Nacht bei 4°C geschüttelt. Anschließend werden die Stents mit Puffer gespült.

### Beispiel 3: Aptamerbeschichtung für einen Stentgrundkörper aus welcher Aptamer im Wesentlichen nicht freigesetzt werden kann

Ein im Sauerstoffplasma (Gerät der Fa. Diener; Modell Tetra; 200 W; 0,2 mBar; 2-30 Min.) oder durch Spülen mit der Lösungsmittelreihe Dichlormethan, Aceton, Methanol und Millipore-Wasser gereinigter Stent wird im Folgenden weiterbehandelt:

Es wird eine 3 mM Lösung von 3-(4-Oxybenzophenon)propylphosphonsäure in trockenem Tetrahydrofuran hergestellt. Mit dieser wird der Stent 3x besprüht.

Anschließend wird der Stent 12 h bei 120°C getempert und daraufhin im Lösungsmittel gespült.

Diese Stents werden in eine Lösung zu der oben beschriebenen erfindungsgemäß einzusetzenden Aptameren (ca. 50 µg/ml) in PBS-Puffer (aminosäurefrei, RNAse-frei) gegeben und über Nacht bei 4°C geschüttelt.

Am nächsten Tag werden die Stents aus dem Lösungsmittel entfernt, getrocknet und bei 360 nm mit 100 mW/cm² belichtet. Nicht gebundene RNA wird abgewaschen.

### Beispiel 4: Aptamerbeschichtung für einen Stentgrundkörper aus welcher Aptamer im Wesentlichen nicht freigesetzt werden kann

Ein im Sauerstoffplasma (Gerät der Fa. Diener; Modell Tetra; 200 W; 0,2 mBar; 2-30 Min.) oder durch Spülen mit der Lösungsmittelreihe Dichlormethan, Aceton, Methanol und Millipore-Wasser gereinigter Stent wird im Folgenden weiterbehandelt:
Es wird eine 1 mM Lösung von Carboxydodecylphosphonsäure in trockenem Tetrahydrofuran hergestellt. In diese wird der Stent gehängt und das Lösungsmittel wird innerhalb von einer Stunde abgedampft, wobei der Meniskus der Lösung über die Stentoberfläche abwandert. Anschließend wird der Stent 74 h bei 120°C getempert und daraufhin im Lösungsmittel gespült. Der so vorbehandelte Stent wird 45 min in eine 1:1 Mischung von 0.4 M EDC und 0.1 M NHS in Millipore Wasser gelegt. Anschließend wird er kurz mit Millipore Wasser gespült und im Stickstoffstrom getrocknet.

Auf die so behandelten Stents wird eine Lösung der oben beschriebenen erfindungsgemäß einzusetzenden Aptameren (ca. 50 µg/ml), in PBS-Puffer (aminosäurefrei, RNAse-frei) gegeben und über Nacht bei 4°C geschüttelt. Anschließend werden die Stents mit Puffer gespült.

## Patentansprüche

1. Implantat für einen menschlichen oder tierischen Körper, **dadurch gekennzeichnet, dass** ein Implantatgrundkörper eine vollständige oder teilweise Beschichtung mit einem oder mehreren, gleichen oder verschiedenen Aptameren umfasst, wobei das oder die Aptamere eine aktivierende Wirkung gegenüber dem αᵥβ₃-Integrin-Rezeptor aufweisen.

2. Implantat gemäß Anspruch 1, **dadurch gekennzeichnet, dass** der Implantatgrundkörper ausgewählt wird aus der Gruppe bestehend aus: Herzschrittmacher, Herzimplantat, Schrittmacherelektrode, Defibrillationselektrode, Shunts, biodegradierbare oder dauerhafte, koronare oder periphere Stents und Neurostents.

3. Implantat gemäß Anspruch 1 oder 2, **dadurch gekennzeichnet, dass** das oder die Aptamere Nukleinsäuren von Aptameren umfassen oder daraus, die an den αᵥβ₃-Integrin-Rezeptor binden, aber eine Endothelzelladhäsion inhibieren und ein oder mehrere Phosphatgruppen des Zucker-phosphatrückgrats zu ein oder mehreren, gleichen oder verschiedenen Thiophosphat- und/oder Methylphosphatgruppen derivatisiert sind.

4. Implantat gemäß einem der Ansprüche 1 bis 3, **dadurch gekennzeichnet, dass** die Beschichtung auf der ganzen oder einem Teil der Oberfläche des Implantatgrundkörpers und/oder in Kavität(en) des Implantatgrundkörpers vorliegen kann.

5. Implantat gemäß einem der Ansprüche 1 bis 4, **dadurch gekennzeichnet, dass** die Beschichtung auf der abluminalen Seite des Implantats vorliegt.

6. Implantat gemäß einem der Ansprüche 1 bis 5, **dadurch gekennzeichnet, dass** die Aptamere unabhängig voneinander unter physiologischen Bedingungen aus der Beschichtung (i) freigesetzt oder (ii) im Wesentlichen nicht freigesetzt werden können.

7. Implantat gemäß Anspruch 6, **dadurch gekennzeichnet, dass** das oder die Aptamere, die (i) aus der Beschichtung freigesetzt werden können, in einer Beschichtung vorliegen, die hydrophobe, biodegradierbare Polymere umfasst oder daraus besteht.

8. Implantat gemäß Anspruch 6, **dadurch gekennzeichnet, dass** das oder die Aptamere, die (ii) im Wesentlichen nicht aus der Beschichtung freigesetzt werden können, unabhängig voneinander über ein oder mehrere, gleiche oder verschiedene Ankergruppen an die Oberfläche des Implantatgrundkörpers gebunden sind.

9. Implantat gemäß einem der Ansprüche 1 bis 8, **dadurch gekennzeichnet, dass** das Implantat zusätzlich ein oder mehrere, gleiche oder verschiedene Wirkstoffe umfasst ausgewählt aus der Gruppe bestehend aus: Antiphlogistika, vorzugsweise Dexamethason, Methylprednisolon und Diclophenac; Cytostatika, vorzugsweise Paclitaxel, Colchicin, Actinomycin D und Methotrexat; Immunsuppressiva, vorzugsweise Limus-Verbindungen, weiter bevorzugt Sirolimus (Rapamycin), Zotarolimus (Abt-578), Tacrolimus (FK-506), Everolimus, Biolimus, insbesondere Biolimus A9 und Pimecrolimus, Cyclosporin A und Mycophenolsäure; Thrombozytenaggregationshemmer, vorzugsweise Abciximab und Iloprost; Statine, vorzugsweise Simvastatin, Mevastatin, Atorvastatin, Lovastatin, Pitavastatin und Fluvastatin; und Estrogene, vorzugsweise 17b-Estradiol, Daizein und Genistein; Lipidregulatoren, vorzugsweise Fibrate; Immunsuppressiva; Vasodilatatoren, vorzugsweise Satane; Calciumkanalblocker; Calcineurininhibitoren, vorzugsweise Tacrolimus; Antiinflammatorika, vorzugsweise Imidazole; Antiallergika; Oligonucleotide, vorzugsweise Decoy-Oligodesoxynukleotid (dODN); Endothelbildner, vorzugsweise Fibrin; Steroide; Proteine/Peptide; Proliferationshemmer; Analgetika und Antirheumatika.

10. Verfahren zur Herstellung eines Implantates gemäß einem der Ansprüche 1 bis 9, **dadurch gekennzeichnet, dass** das Verfahren folgende Schritte umfasst oder daraus besteht:
a) Bereitstellen eines Implantatgrundkörpers,
b) Bereitstellen eines Beschichtungsmaterials umfassend ein oder mehrere, gleiche oder verschiedene Aptamere, die eine aktivierende Wirkung gegenüber dem αᵥβ₃-Integrin-Rezeptor aufweisen, wobei das Beschichtungsmaterial geeignet ist, das oder die Aptamere unter physiologischen Bedingungen aus der Beschichtung freizusetzen und
c) Aufbringen des Beschichtungsmaterials aus Schritt b) auf mindestens einen Teil der Oberfläche und/oder in Kavität(en) des Implantatgrundkörpers.

11. Verfahren zur Herstellung eines Implantates gemäß einem der Ansprüche 1 bis 9, **dadurch gekennzeichnet, dass** das Verfahren folgende Schritte umfasst oder daraus besteht:
a) Bereitstellen eines Implantatgrundkörpers,
b) Bereitstellen einer oder mehrerer, gleicher oder verschiedener Ankergruppen,
c) Funktionalisieren des Implantatgrundkörpers aus Schritt a) mit der oder den Ankergruppen aus Schritt b),
d) Bereitstellen eines oder mehrerer, gleicher oder verschiedener Aptamere, die eine aktivierende Wirkung gegenüber dem αᵥβ₃-Integrin-Rezeptor aufweisen und
e) Binden des oder der Aptamere aus Schritt d) an den oder die funktionalisierten Implantatgrundkörper aus Schritt c).

12. Verwendung eines Aptamers mit aktivierender Wirkung gegenüber dem αᵥβ₃-Integrin-Rezeptor zur Vermittlung Endothelzelladhäsion an ein Implantat für einen menschlichen oder tierischen Körper.

13. Verwendung eines oder mehrerer unterschiedlicher Aptamere mit aktivierender Wirkung gegenüber dem αᵥβ₃-Integrin-Rezeptor zur vollständigen oder teilweisen Beschichtung eines Implantatgrundkörpers für einen menschlichen oder tierischen Körper.

14. Verwendung eines oder mehrerer unterschiedlicher Aptamere mit aktivierender Wirkung gegenüber dem αᵥβ₃-Integrin-Rezeptor zur Herstellung eines Implantats gemäß einem der Ansprüche 1 bis 9.


Sequenzprotokoll

<110> BIOTRONIK VI Patent AG

<120> Aptamer beschichtetes Implantat, Herstellverfahren und Verwendungen

<130> 09.151P-EP

<160> 1

<170> PatentIn version 3.3

<210> 1
<211> 80
<212> DNA
<213> Künstliche Sequenz

<220>
<223> note:"Beschreibung der künstlichen Sequenz: Sequenz bindet an alpha v
beta 3 Integrin-Receptor"

<400> 1
catgcgcctt ccccctgtgg ttggtgtcag tcggcctgtg tgattagtgg aatgtgcgat 60

gggtctccca gcgggattgg 80